# EUROPEAN PATENT APPLICATION

(11) **EP 4 549 565 A1**
(43) Date of publication of application: **07.05.2025**
(21) Application number: 23831625.1
(22) Date of filing: 30.06.2023
(51) Int. Cl.: C12N 15/09, C12N 15/11

(54) **TRACRRNA UNIT AND GENOME EDITING METHOD**

(30) Priority: 30.06.2022 JP 2022106658
(71) Applicant: Regional Fish Institute, Ltd., Kyoto-shi, Kyoto 606-8501 (JP)
(72) Inventor: KISHIMOTO, Kenta, Kyoto-shi, Kyoto 606-8501 (JP); KUNII, Atsushi, Kyoto-shi, Kyoto 606-8501 (JP)
(74) Representative: Winter, Brandl - Partnerschaft mbB
(86) International application number: PCT/JP2023/024367
(87) International publication number: WO 2024/005186

(57) **Abstract**

An object of the present invention is to provide a novel tracrRNA unit and a genome editing method using this unit.

The objects are achieved by a tracrRNA unit comprising a first single-stranded RNA and a second single-stranded RNA for use in a CRISPR-Cas9 genome editing system wherein the first single-stranded RNA and the second single-stranded RNA are not continuous, the first single-stranded RNA has at least a first segment and a second segment, the first and second segments do not overlap, the first segment has a sequence complementary to a part of a crRNA, and the second segment has a sequence complementary to the second single-stranded RNA.

## Description

### Technical Field

The present description discloses a tracrRNA unit and a genome editing method.

### Background Art

Clustered regularly interspaced short palindromic repeats/CRISPR associated protein 9(CRISPR/Cas9) system is an immune system against viruses and plasmids found in bacteria and archaea (Non Patent Literature 1). This system is currently used in vitro as a method for deleting, replacing, or inserting nucleotide sequences into target regions in genomic DNA of various organisms (Patent Literatures 1 and 2).

In bacteria and archaea, a CRISPR RNA (crRNA) having a nucleotide sequence complementary to a nucleotide sequence of a region of interest in genomic DNA and a tracrRNA having a nucleotide sequence complementary to the crRNA are separate RNA strands, which hybridize via the nucleotide sequences that are complementary to each other to construct a crRNA:tracrRNA duplex to guide a Cas9. On the other hand, as shown in Non Patent Literature 1, Patent Literature 1, and Patent Literature 2, a single guide RNA (sgRNA) where the crRNA and the tracrRNA are linked as a single RNA strand with a linker loop sequence in between is generally used in vitro instead of the crRNA:tracrRNA duplex.

### Citation List

### Patent Literature

Patent Literature 1: WO2013176772A1
Patent Literature 2: WO2014093661A2

### Non Patent Literature

Non Patent Literature 1: M Jinek, et al.:SCIENCE, 28 Jun 2012,Vol 337, Issue 6096, pp. 816-821
Non Patent Literature 2: H Nishimasu, et al.:Cell, 27 Feb 2014, Vol 156, pp. 935-949

### Summary of Invention

### Technical Problem

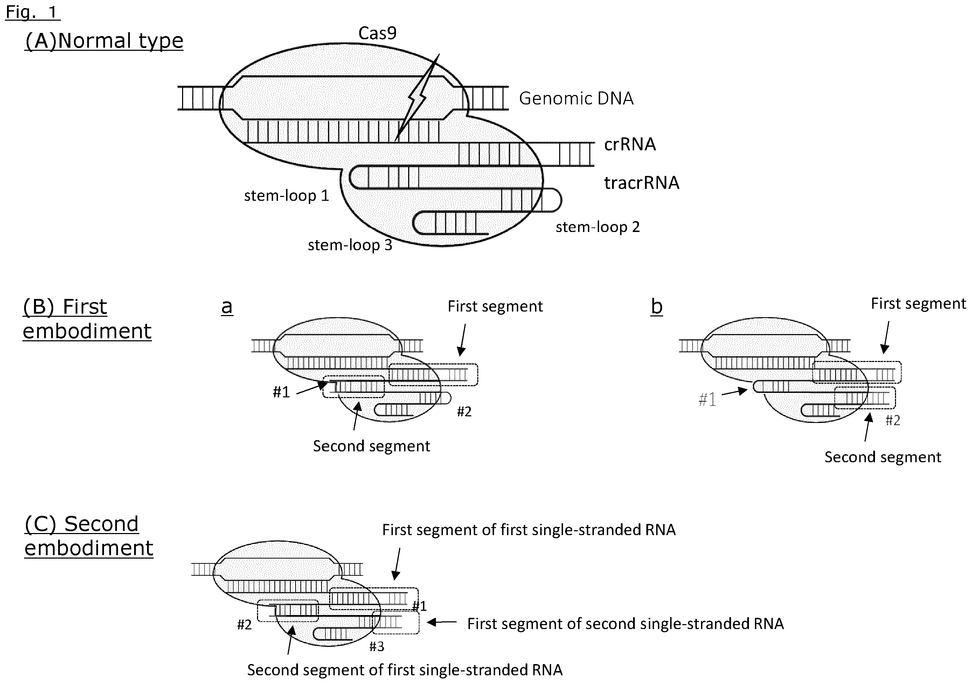

The tracrRNA constructs three hairpin loops within its RNA strand, as shown in Fig. 1A. The three hairpin loops are referred to as a first stem-loop, a second stem-loop, and a third stem-loop in order of proximity to a nucleotide sequence that hybridizes with the crRNA. With regard to the tracrRNA, it is known that it can function as tracrRNA even when the linker segment between the first and second stem-loops is deleted, as described in Non-Patent Literature 2, but it is completely unclear whether it can maintain its function as tracrRNA when the other regions are modified.

An object of the present invention is to provide a novel tracrRNA unit and a genome editing method using the tracrRNAunit.

### Solution to Problem

### Item 1.

A tracrRNA unit comprising a first single-stranded RNA and a second single-stranded RNA for use in a CRISPR-Cas9 genome editing system wherein
the first single-stranded RNA and the second single-stranded RNA are not continuous,
the first single-stranded RNA has at least a first segment and a second segment, and
the first and second segments do not overlap, the first segment has a sequence complementary to a part of a crRNA, and the second segment has a sequence complementary to the second single-stranded RNA.

### Item 2.

A tracrRNA unit comprising a first single-stranded RNA, a second single-stranded RNA, and a third single-stranded RNA for use in a CRISPR-Cas9 genome editing system wherein
the first single-stranded RNA, the second single-stranded RNA, and the third single-stranded RNA are not continuous,
the first single-stranded RNA has at least a first segment and a second segment,
the first and second segments do not overlap, the first segment has a sequence complementary to a part of a crRNA, and the second segment has a sequence complementary to the second single-stranded RNA,
the second single-stranded RNA has at least a first segment, and
the first segment of the second single-stranded RNA has a sequence complementary to the third single-stranded RNA.

### Item 3.

The tracrRNA unit according to item 1 or 2, wherein the crRNA and the first single-stranded RNA are linked in order.

### Item 4.

The tracrRNA unit according to item 2, wherein the crRNA, the first single-stranded RNA and the second single-stranded RNA are linked in order.

### Item 5.

The tracrRNA unit according to item 1, wherein at least one of the first single-stranded RNA and the second single-stranded RNA has a length of 39 nucleotides or less.

### Item 6.

The tracrRNA unit according to item 2, wherein at least one of the first single-stranded RNA, the second single-stranded RNA, and the third single-stranded RNA has a length of 39 nucleotides or less.

### Item 7.

The tracrRNA unit according to item 2, wherein the first single-stranded RNA and the second single-stranded RNA are not continuous, and wherein the second single-stranded RNA and the third single-stranded RNA are continuous.

### Item 8.

A genome editing method using
the tracrRNA unit according to any one of items 1 to 7,
a crRNA and
a Cas9.

### Advantageous Effects of Invention

A novel tracrRNA unit and a genome editing method using this can be provided. As each RNA strand of the novel tracrRNA unit is short, cost of chemically synthesising the RNA strands can be reduced. In addition, by splitting the tracrRNA, the number of 5' and 3' ends of the RNA strands can be increased, and the number and variety of functional motifs added to the tracrRNA can be increased, thereby increasing applications for donor tethering and designs of general nucleic acid modifications.

### Brief Description of Drawings

Fig. 1 shows a structure of a normal type of tracrRNA and an overview of a tracrRNA unit of the present invention. Fig. 1A shows a structure of a normal type of crRNA:tracrRNA duplex. Fig. 1B shows a structure of a tracrRNA unit according to a first embodiment of the present invention. Fig. 1B(a) shows a construct in which the tracrRNA is split at a first stem-loop. Fig. 1B(b) shows a construct in which the tracrRNA is split at a second stem-loop. Fig. C shows a structure of a tracrRNA unit according to a second embodiment of the present invention.
Fig. 2 shows a sequence of the normal type of tracrRNA.
Fig. 3 shows one embodiment of the tracrRNA unit.
Fig. 4 shows one embodiment of the tracrRNA unit.
Fig. 5 shows one embodiment of the tracrRNA unit.
Fig. 6 shows one embodiment of the tracrRNA unit.
Fig. 7 shows a result of an in vitro cleavage assay targeting a slc45a2 gene of Japanese rice fish, medaka (Oryzias latipes).
Fig. 8 shows observation results of eyes of medaka embryos injected with a tracrRNA consisting of a first single-stranded RNA and a second single-stranded RNA or a tracrRNA consisting of the first single-stranded RNA, the second single-stranded RNA, and a third single-stranded RNA, together with a Cas9 and a crRNA.
Fig. 9 shows results of analysis by HMA using lysates of the medaka embryos injected with the tracrRNA consisting of the first single-stranded RNA and the second single-stranded RNA or the tracrRNA consisting of the first single-stranded RNA, the second single-stranded RNA, and the third single-stranded RNA, together with the Cas9 and the crRNA.
Fig. 10-1 shows a result of mutation analysis by a Sanger sequencing method and a TIDE.
Fig. 10-2 shows a result of mutation analysis by the Sanger sequencing method and the TIDE.
Fig. 10-3 shows a result of mutation analysis using the Sanger sequencing method and the TIDE.
Fig. 10-4 shows a result of mutation analysis using the Sanger sequencing method and the TIDE.
Fig. 10-5 shows a result of mutation analysis using the Sanger sequencing method and the TIDE.

### Description of Embodiments

### 1. tracrRNA unit

In the present description, a tracrRNA unit is intended to be a complex in which a tracrRNA is comprised of two or more single-stranded RNAs and maintains its function as a tracrRNA.

### Notation of a nucleotide follows common notation of RNA or DNA.

In the present description, "linked" is intended to be linked as a single polynucleotide chain. Preferably, it is intended that two polynucleotide chains are linked by phosphodiester bonds.

In the present description, "having a sequence" is intended to contain or consist of the sequence.

### 1-1. First embodiment

A first embodiment relates to a tracrRNA unit in which the tracrRNA is split into two segments in a first or second stem-loop shown in Figure 1B. In Fig. 1B(a), the tracrRNA is split in the first stem-loop. In Fig. 1B(b), the tracrRNA is split in the second stem-loop.

More specifically, the tracrRNA unit can be comprised of the first single-stranded RNA (represented by "#1" in Fig. 1B) and the second single-stranded RNA (represented by "#2" in Fig. 1B). The first and second single-stranded RNAs are not continuous. The first single-stranded RNA has at least a first segment and a second segment, and the first segment and the second segment do not overlap. The first segment has a sequence complementary to a part of a crRNA, and the second segment has a sequence complementary to the second single-stranded RNA.

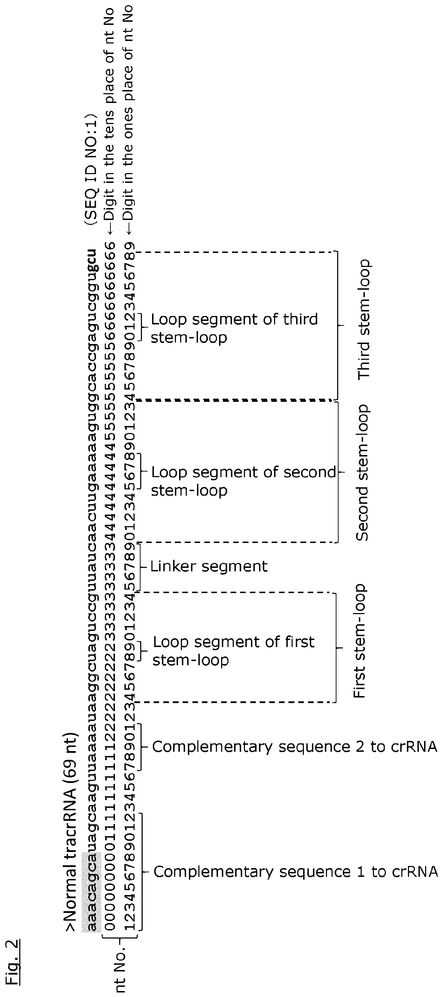

Fig. 2 shows a nucleotide sequence (SEQ ID NO: 1) of a normal type of tracrRNA. In the normal type of tracrRNA, nucleotide numbers (nt No.) 1 to 12 are a first complementary sequence to the crRNA, nt Nos. 17 to 21 are a second complementary sequence to the crRNA, nt Nos. 28 to 29 are a loop segment of the first stem-loop, nt Nos. 35 to 39 are a linker segment, nt Nos. 45 to 48 are a loop segment of the second stem-loop, nt Nos. 60 to 62 are a loop segment of a third stem-loop. Sequences before and after each loop segment hybridize to construct a hairpin loop. For example, the first stem-loop is constructed by hybridization of at least nt Nos. 25 to 27 and nt Nos. 33, 32, and 30. The second stem-loop is constructed by hybridization of at least nt Nos. 41 to 44 and nt Nos. 52 to 49. The third stem-loop is constructed by hybridization of at least nt Nos. 54 to 59 and nt Nos. 68 to 63.

When the tracrRNA is split in the first stem-loop, it can be split between nt Nos. 27 and 28, between nt Nos. 28 and 29, or between nt Nos. 29 and 30 of SEQ ID NO: 1. Alternatively, it may be split by deleting nt No. 28, nt No. 29, and nt No. 28 and 29.

When the tracrRNA is split in the second stem-loop, it can be split between nt Nos. 44 and 45, between nt Nos. 45 and 46, between nt Nos. 46 and 47, between nt Nos. 47 and 48, or between nt Nos. 48 and 49 of SEQ ID NO: 1. Alternatively, it may be split by deleting one or more nucleotides in nt No. 45 to 48.

The first single-stranded RNA and/or the second single-stranded RNA may use an RNA with a split nucleotide sequence in its sequence. Alternatively, an additional sequence of about 1 to 10 nucleotides may be added to one or both of the 5' end and the 3' end of the first single-stranded RNA and/or the second single-stranded RNA.

The first single-stranded RNA and/or the second single-stranded RNA may be partially deleted as long as it functions as the tracrRNA when constructed as the tracrRNA unit. In this case, the length of at least one of the first single-stranded RNA and the second single-stranded RNA is preferably 39 nucleotides or less. When the first single-stranded RNA and the second single-stranded RNA contain the additional sequence, the length of at least one of the first single-stranded RNA and the second single-stranded RNA containing the additional sequence is preferably 39 nucleotides or less.

In this case, the part of the first segment of the first single-stranded RNA that is complementary to a part of the crRNA should comprise a part of at least one of the first complementary sequence to the crRNA and the second complementary sequence to the crRNA, as shown in Fig. 2. In addition, the part that is complementary to the second single-stranded RNA in the second segment of the first single-stranded RNA should also contain a part of sequences before and after the loop segment of the first stem-loop, or a part of sequences before and after the loop segment of the second stem-loop.

In SEQ ID NO: 1, as described in Non-Patent Literature 2, the function of the tracrRNA is maintained even when the linker segment of nt Nos. 35 to 39 is partially or entirely, preferably, nt Nos. 36 to 39 is deleted. Therefore, an aspect in which only nt Nos. 36 to 39, preferably only the linker segment, is deleted is preferably excluded from this embodiment.

In this embodiment, for example, the tracrRNA can function as the tracrRNA or as the tracrRNA unit even when nt Nos. 1 to 8 of SEQ ID NO: 1 is deleted.

In this embodiment, the first single-stranded RNA may be linked to the crRNA described below.

In the case that a part of the nucleotide sequence of the tracrRNA is deleted, the first single-stranded RNA and the second single-stranded RNA may be linked in order from the 5' side to the 3' side to construct the tracrRNA unit.

The first single-stranded RNA and the second single-stranded RNA are preferably annealed between complementary sequences to construct the tracrRNA unit before being introduced into cells, individuals or tissues. The annealing is not particularly limited, but is achieved, for example, by heating at about 90°C to 98°C for about 3 to 10 minutes in a thermal cycler or the like, and then gradually lowering the temperature to room temperature (e.g., 0.1°C per second down to 25°C). At the time of the annealing, the crRNA may also be added and annealed to construct a guide RNA unit.

### 1-2. Second embodiment

A second embodiment relates to a tracrRNA unit in which the tracrRNA is split into three segments in a first stem-loop and a second stem-loop shown in Figure 1C. In Fig. 1C, the tracrRNA is split in the first stem-loop and the second stem-loop.

More specifically, the tracrRNA unit can be comprise of a first single-stranded RNA (represented by "#1" in Fig. 1C), a second single-stranded RNA (represented by "#2" in Fig. 1C) and a third single-stranded RNA (represented by "#3" in Fig. 1C). The first, second, and third single-stranded RNAs are not continuous. The first single-stranded RNA has at least a first segment and a second segment, the first segment and the second segment do not overlap, the first segment has a sequence complementary to a part of the crRNA, and the second segment has a sequence complementary to the second single-stranded RNA. The second single-stranded RNA has at least a first segment, and the first segment of the second single-stranded RNA has a sequence complementary to the third single-stranded RNA. Here, in the second single-stranded RNA, the part that is complementary to the second segment of the first single-stranded RNA does not overlap with the first segment of the second single-stranded RNA.

Since the splitting in the first stem-loop and the second stem-loop is the same as in the first embodiment, the above description is incorporated herein.

When the tracrRNA is split in the third stem-loop, it can be split between nt Nos. 59 and 60, between nt Nos. 60 and 61, between nt Nos. 61 and 62, or between nt Nos. 62 and 63 of SEQ ID NO: 1. Alternatively, it may be split by deleting one or more nucleotides in nt No. 60 to 62.

At least one of the first single-stranded RNA, the second single-stranded RNA, and the third single-stranded RNA may use an RNA with a split nucleotide sequence in its sequence. Alternatively, at least one of the first single-stranded RNA, the second single-stranded RNA, and the third single-stranded RNA may have an additional sequence of about 1 to 10 nucleotides at one or both of the 5' end and the 3' end.

At least one of the first single-stranded RNA, the second single-stranded RNA, and the third single-stranded RNA may be partially deleted as long as it functions as the tracrRNA when constructed as the tracrRNA unit. In this case, the length of at least one of the first single-stranded RNA, the second single-stranded RNA, and the third single-stranded RNA, including the additional sequence, is preferably 39 nucleotides or less.

In this case, the part that is complementary to a part of the crRNA in the first segment of the first single-stranded RNA should comprise a part of at least one of the first complementary sequence to the crRNA and the second complementary sequence to the crRNA, as shown in Fig. 2. In addition, the portion that is complementary to the second single-stranded RNA in the second segment of the first single-stranded RNA should also contain a part of sequences before and after the loop segment of the first stem-loop, or a part of sequences before and after the loop segment of the second stem-loop. Furthermore, the part that is complementary to the third single-stranded RNA in the second single-stranded RNA should also contain a part of the sequences before and after the loop segment of the third stem-loop.

In SEQ ID NO: 1, as described in Non-Patent Literature 2, the function of the tracrRNA is maintained even when the linker segment of nt Nos. 35 to 39 is deleted. Therefore, preferably, an aspect in which only the linker segment is deleted is excluded from this embodiment.

In this embodiment, for example, the tracrRNA can function as the tracrRNA or as the tracrRNA unit even when nt Nos. 1 to 8 of SEQ ID NO:1 is deleted.

In this embodiment, the first single-stranded RNA may be linked to the crRNA. The crRNA contains a nucleotide sequence (preferably 20 nucleotides) complementary to a target genomic sequence and a nucleotide sequence complementary to the first single-stranded RNA. Furthermore, the first single-stranded RNA linked to the crRNA may be linked to the second single-stranded RNA.

In the case that a part of the nucleotide sequence of the tracrRNA is deleted, the first single-stranded RNA, the second single-stranded RNA, and the third single-stranded RNA may be linked in order from the 5' side to the 3' side to construct the tracrRNA unit.

The first single-stranded RNA, the second single-stranded RNA, and the third single-stranded RNA are preferably annealed between complementary sequences to construct the tracrRNA unit before being introduced into the cell, individual or tissue. The annealing is not particularly limited, but is achieved, for example, by heating at about 90°C to 98°C for about 3 to 10 minutes in the thermal cycler or the like, and then gradually lowering the temperature to room temperature (e.g., 0.1°C per second down to 25°C). At the time of the annealing, the crRNA may also be added and annealed to construct the guide RNA unit.

### 2. Chimeric RNA obtained by linking crRNA and tracrRNA unit

A third embodiment relates to a chimeric RNA obtained by linking the crRNA and the single-stranded RNA constituting the tracrRNA unit described in the above section 1. The chimeric RNA lacks a part of the nucleotide sequence shown in SEQ ID NO: 1.

### 3. DNA fragment encoding each RNA constituting tracrRNA unit or encoding chimeric RNA

A fourth embodiment relates to a DNA fragment encoding each single-stranded RNA described in the above section 1. or the chimeric RNA described in the above section 2.

The DNA fragment may be single-stranded or double-stranded.

In addition, the DNA fragment may be incorporated into a vector. The vector is not limited as long as it can transcribe the above single-stranded RNA or chimeric RNA from the DNA fragment. The DNA fragment is inserted downstream of a nucleotide sequence of a promoter such as H1, 7SK, and U6 promoter. Examples of the vectors can include plasmid vectors, adenovirus vectors, adeno-associated virus vectors, retrovirus vectors, lentivirus vectors, and the like.

### 4. Genome editing method using tracrRNA unit

A fifth embodiment relates to a genome editing method using the tracrRNA unit described in the above section 1. The fifth embodiment also relates to a genome editing method using the chimeric RNA as described in the above section 2. The genome editing can include modes of substitution, deletion and insertion in a nucleotide sequence of the target genome, or techniques such as gene expression control and epigenome editing.

Here, "use"' is intended to mean that the tracrRNA unit and/or the chimeric RNA are used for the genome editing in a cell. However, it does not matter whether it is introduced into the cell in the construct of RNA or the construct of the DNA fragment incorporated in the vector as described in the above section 3.

A preferred aspect is to use the tracrRNA unit described in the above section 1 together with the crRNA and a CRISPR (Clustered Regularly Interspaced Short Palindromic Repeats) enzyme.

The crRNA contains the nucleotide sequence (preferably 20 nucleotides) complementary to the target genomic sequence (hereinafter, also referred to as "target sequence") and a nucleotide sequence complementary to all or part of the first segment of the first single-stranded RNA described above.

A method for selecting the target sequence is known. The targeting sequence may be selected using a known design tool published in Optimized CRISPR design tool (web page of Massachusetts Institute of Technology, ZhangLab (http://crispr.mit.edu/)), E-CRISP (http://www.e-crisp.org/E-CRISP/ (German Cancer Research Center)), ZiFiT Targeter(http://zifit.partners.org/ZiFit/ (Zing Finger consortium)), Cas9 design (http://cas9.cbi.pku.edu.cn (Peking University)), CRISPRdirect (http://crispr.dbcls.jp (University of Tokyo)), CRISPR-P (http://cbi.hzau.edu.cn/crispr/ (Huazhong Agricultural University)), CRISPR RGEN Tools (http://www.rgenome.net/ (Seoul National University)), and the like.

The tracrRNA unit, crRNA, CRISPR enzyme, and a nucleic acid encoding this enzyme can be introduced by such methods as microinjection, electroporation and lipofection.

The tracrRNA unit and crRNA can be injected in a range of about 0.05 pg to 25 pg, preferably 0.25 pg to 10 pg, more preferably 0.5 pg to 2.5 pg, respectively.

When the CRISPR enzyme is introduced as a protein or a nucleic acid encoding the protein, it can be injected in a range of about 5 pg to 100 pg, preferably 10 pg to 80 pg, more preferably 10 pg to 50 pg per cell.

The tracrRNA unit, crRNA, CRISPR enzyme or nucleic acid encoding this enzyme may be administered systemically or topically to an individual using a delivery system including liposomes. For systemic administration, the tracrRNA unit, crRNA, CRISPR enzyme or nucleic acid encoding this enzyme can be administered at a dose of 0.1 to 1,000 mg/day per kg body weight of an individual, respectively. For topical administration, the tracrRNA unit, crRNA, CRISPR enzyme or nucleic acid encoding this enzyme can be administered at a dose of 0.01 to 100 mg/day per cm² of target tissue, respectively.

Alternatively, instead of using the tracrRNA unit and crRNA as independent units, the tracrRNA unit and crRNA may be linked together and used as the chimeric RNA. The chimeric RNA can be injected in a range of 0.1 pg to 50 pg, preferably 0.5 pg to 20 pg, more preferably 1 pg to 5 pg per cell. When the chimeric RNA is administered systemically, it can be administered at a dose of 0.1 to 1,000 mg/day per kg body weight of an individual. When the chimeric RNA is administered topically, it can be administered at a dose of 0.01 to 100 mg/day per cm² of target tissue.

The tracrRNA unit, crRNA, or chimeric RNA may be used by introducing the vector incorporating the DNA fragment encoding these RNAs described in the above section 3 into the cell, individual, or tissue, and expressing the RNA encoded in the DNA fragment after the introduction.

If the vector is the plasmid vector, it can be introduced in a range of about 5 pg to 100 pg, preferably 10 pg to 80 pg, more preferably 10 pg to 50 pg as a total amount of DNA per cell. When the plasmid vector is administered systemically, it can be administered at a dose of 0.1 to 1,000 mg/day as a total amount of DNA per kg body weight of an individual. When the plasmid vector is administered topically, it can be administered at a dose of 0.01 to 100 mg/day as a total amount of DNA per cm² of target tissue. If the plasmid vector incorporating the DNA encoding the CRISPR enzyme is used, it is also included in the total amount of DNA.

If the vector is the virus vector, it can be introduced in a range of about 10² to 10⁶ vg as a total amount of virus incorporating the target nucleotide sequence per cell. When the virus is administered systemically, it can be administered at a dose of 10¹⁰ to 10¹⁸ vg/day as a total amount of virus per kg body weight of an individual. When the virus is administered topically, it can be administered at a dose of 10⁹ to 10¹⁶ vg/day as a total amount of virus per cm² of target tissue. If the virus incorporating the DNA encoding the CRISPR enzyme is used, it is also included in the total amount of DNA.

Furthermore, in this embodiment, donor oligo DNA such as single-stranded oligodeoxynucleotide (ssODNs) may be co-introduced together with the tracrRNA unit, crRNA, CRISPR enzyme or nucleic acid encoding this enzyme. The ssODNs can be designed according to known methods.

Examples of the CRISPR enzyme can include, for example, Cas 9 derived from Streptococcus pyogenes, Staphylococcus aureus, Francisella novicida, Campylobacter jejuni, Neisseria meningitidis, Streptococcus thermophilus, Clostridium cellulolyticum, Treponema denticola, Brevibacillus laterosporus, Actinobacillus succinogenes, and the like. In addition, other examples of the Cas9 such as Lpn, Khu, Ain, Cgl, Esp1, Esp2, Fma, Lce, Lrh, Lsp1, Lsp2, Pac, Tba, Tde, Tpu, Vpa, Efa, Eit, Lan, Lmo, Sag1, Sag2, Sdy, Seq1, Seq2, Sga, Smu, Sra, Bni, Ece, Edo, Fho, Mga, Mse, Sgo, Sma2, Ssa, Ssi, Ssu, Sth1A, Tsp, Bok, Cco, Cpe, Dde, Ghc2, Ghy3, Ghy4, Gsp, Kki, Nme2, Nsp, Tmo, Nsa, Jpa, Rsp, Bbo, Cca2, Cme2, Cme3, Cme4, Csa, Ghc1, Ghe, Ghh1, Ghh2, Ghy1, Msc, Sdo, Spa, Cca1, Cga, Cme1, Ffr, Ghy2, Orh, Phi, Psp, Wvi, and the like, described in Supplementary Data 2 in literature: Gasiunas et al. (2020), Nat Commun, 11_5512, can be included.

Furthermore, the Cas9 may have an amino acid substitution and/or be bound to an effector molecule. Examples of amino acid-substituted Cas9 can include, a high-specificity mutation such as R691A mutation (also referred to as HiFiCas9) of Streptococcus pyogenesCas9 (also referred to as SpCas9), a nuclease-inactivating mutation such as D10A and H840A mutations (dCas9) of the SpCas9, and the like. The effector molecule can include a Rad51 protein, which promotes homologous recombination repair, and a CtIP protein, which promotes microhomology-mediated end-joining. The effector molecule can be bound to the Cas9 via a linker. A reverse transcriptase can also be bound as the effector to nCas9 with the H840A mutation as the Cas9.

In the present description, "Cas9" can include wild-type Cas9 derived from the various organisms described above, mutant Cas9 with the amino acid substitution, mutant Cas9 bound to the effector molecule, and the like.

The CRISPR enzyme can be introduced into the cell, individual or tissue in the construct of the protein or the nucleic acid encoding the protein.

An organism to which the genome editing method of this embodiment is applied is not particularly limited. The organism may be a prokaryote, a eukaryote or a cultured cell derived from the eukaryotic cellular organism. The eukaryotic cellular organism can include, for example, an animal cellular organism such as fishes, reptiles, amphibians, birds, mammals, insects, crustaceans, arthropods, molluscs, echinoderms, cnidarians, annelids, and nematodes, a plant cellular organism such as plants and algae, fungi, protozoa, and the like.

### 5. Genome editing kit

The fourth embodiment relates to a genome editing kit containing the tracrRNA unit, crRNA and CRISPR enzyme or nucleic acid encoding this enzyme, as described in the above section 1. For explanation of each term, the explanations in the above sections 1 and 4 are incorporated herein.

The fifth embodiment relates to a genome editing kit containing the chimeric RNA and CRISPR enzyme or nucleic acid encoding this enzyme, as described in the above section 2. For explanation of each term, the explanations in the above sections 2 and 4 are incorporated herein.

A sixth embodiment relates to a genome editing kit containing the DNA fragment encoding each RNA constructing the tracrRNA unit or encoding the chimeric RNA, as described in the above section 3, and the CRISPR enzyme or the nucleic acid encoding this enzyme. For explanation of each term, the explanations in the above sections 3 and 4 are incorporated herein.

### 6. Variation

Each RNA strand constructing the tracrRNA unit disclosed herein and the chimeric RNA may be provided with a functional motif at the 5' and/or 3' end. The functional motif can include, for example, biotin labeling, nuclease resistance modification, fluorescent labeling, protein binding motifs, and the like.

### Examples

Hereinafter, aspects of the present invention will be described in more detail with reference to examples. However, the present invention shall not be construed as limited to the examples.

### I. Design of tool for genome editing experiment

### (i) crRNA

In the present description, a nucleotide sequence of a crRNA is, where base pairing with a target DNA is 20 nucleotides, 5'-NNNNNNNNNNNNNGUUUUAGAGCUAUGCUGUUUUG-3' (SEQ ID NO: 2), wherein "N" is an optional nucleotide. In this example, the target gene is slc45a2 of medaka, a target sequence containing PAM is 5'-ACGGAGCGCACTGGAAGCTGAGG-3' (SEQ ID NO: 3), and the nucleotide sequence of the crRNA is 5'-ACGGGAGCGCACUGGGAAGCUGGUUUUAGAGCUAUGCUGUUUUG-3' (SEQ ID NO: 4). Synthesis of the RNA was performed by FASMAC.

### (ii) normal type of tracrRNA

In the present description, a nucleotide sequence of a normal type of tracrRNA is 5'-AAACAGCAUAGCAAGUUAAAUAAGGCUAGUCCGUUAUCAACUUGAAAAAAGUGG CACCGAGUCGGUGCU-3' (SEQ ID NO: 1) shown in Fig. 2, in which nt Nos. 24 to 34 are referred to as a "first stem-loop", nt Nos. 35 to 39 are referred to as a "linker" and nt Nos. 40 to 53 are referred to as a "second stem-loop". Synthesis of the RNA was performed by FASMAC.

### (iii) Shortening of 5' side sequence in tracrRNA

In the normal type of tracrRNA described in the above section (ii), nt Nos. 1 to 8 were deleted. The shortened nucleotide sequence is 5'-UAGCAAGUUAAAUAAGGCUAGUCCGUUAUCAACUUGAAAAAAGUGGCACCGAGU CGGUGCU-3' (SEQ ID NO: 5). Synthesis of the RNA was performed by FASMAC.

### (iv) Shortening of linker in tracrRNA

In the normal type of tracrRNA described in the above section (ii), nt Nos. 36 to 39 were deleted. The shortened nucleotide sequence is 5'-AAACAGCAUAGCAAGUUAAAAUAAGGCUAGUCCGUAACUUGAAAAAGUGGCACC GAGUCGGUGCU-3' (SEQ ID NO: 6). Synthesis of the RNA was performed by FASMAC.

### (v) Splitting of first stem-loop in tracrRNA

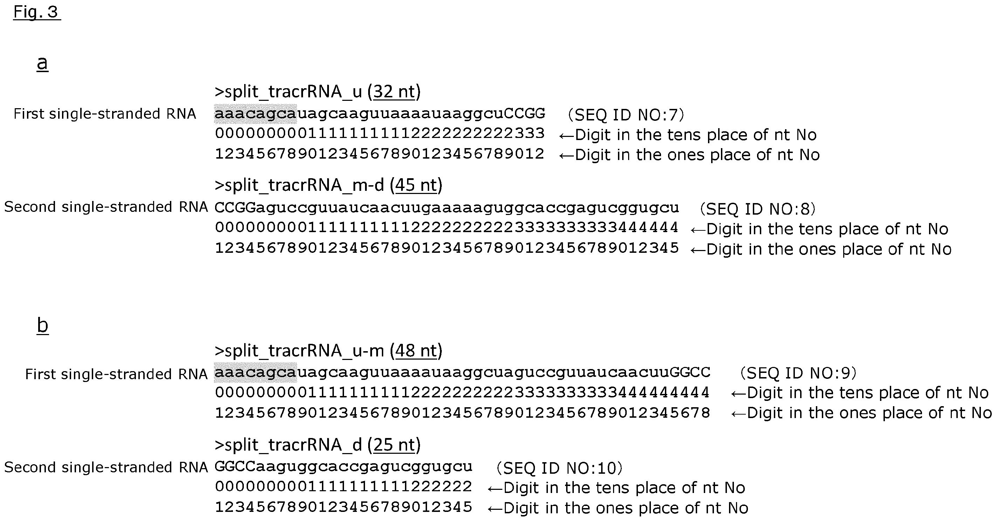

The normal type of tracrRNA described in the above section (ii) was split into nt Nos. 1 to 28 and nt Nos. 29 to 69, and 5'-CCGG-3' was added to a 3' side of a first single-stranded RNA located on the 5' side, and 5'-CCGG-3' was added to a 5' side of a second single-stranded RNA located on the 3' side. A sequence of a unit is shown in Fig. 3a. The nucleotide sequence of the first single-stranded RNA is 5'-AAACAGCAUAGCAAGUUAAAAUAAGGCUCCGG-3' (SEQ ID NO: 7), and the nucleotide sequence of the second single-stranded RNA is 5'-CCGGAGUCCGUUAUCAACUUGAAAAAGUGGCACCGAGUCGGUGCU-3' (SEQ ID NO: 8). By Watson-Crick base pairing, it is expected that nt Nos. 25, 26, 27, 28, 29, 30, 31, 32 of the first single-stranded RNA represented by SEQ ID NO: 7 in Fig. 3a will interact with nt Nos. 9, 8, 6, 5, 4, 3, 2, 1 of the second single-stranded RNA represented by SEQ ID NO: 8 in Fig. 3a, and by mismatch base pairing, it is expected that nt No. 24 of the first single-stranded RNA represented by SEQ ID NO: 7 in Fig. 3a will interact with nt No. 10 of the second single-stranded RNA represented by SEQ ID NO: 8 in Fig. 3a. The first "a" in SEQ ID NO: 7 corresponds to the first "a" in SEQ ID NO: 1 shown in Fig. 2, and the fifth "a" in SEQ ID NO: 8 corresponds to the 29th "a" in SEQ ID NO: 1 shown in Fig. 2. Synthesis of the RNA was performed by FASMAC.

### (vi) Splitting of second stem-loop in tracrRNA

The normal type of tracrRNA described in the above section (ii) was split into nt Nos. 1 to 44 and nt Nos. 49 to 69, 5'-GGCC-3' was added to a 3' side of a first single-stranded RNA located on the 5' side, and 5'-GGCC-3' was added to a 5' side of a second single-stranded RNA located on the 3' side. A sequence of a unit is shown in Fig. 3b. The nucleotide sequence of the first single-stranded RNA represented by SEQ ID NO: 9 in Fig. 3b is 5'-AAACAGCAUAGCAAGUUAAAAUAAGGCUAGUCCGUUAUCAACUUGGCC-3' (SEQ ID NO: 9), and the nucleotide sequence of the second single-stranded RNA represented by SEQ ID NO: 10 in Fig. 3b is 5'-GGCCAAGUGGCACCGAGUCGGUGCU-3' (SEQ ID NO: 10). By Watson-Crick base pairing, it is expected that nt Nos. 41, 42, 43, 44, 45, 46, 47, 48 of the first single-stranded RNA will interact with nt Nos. 8, 7, 6, 5, 4, 3, 2, 1 of the second single-stranded RNA, and by mismatch base pairing, it is expected that nt No. 40 of the first single-stranded RNA will interact with nt No. 9 of the second single-stranded RNA. The first "a" in SEQ ID NO: 9 corresponds to the first "a" in SEQ ID NO: 1 shown in Fig. 2, and the fifth "a" in SEQ ID NO: 10 corresponds to the 49th "a" in SEQ ID NO: 1 shown in Fig. 2. Synthesis of the RNA was performed by FASMAC.

### (vii) Shortening of 5' side sequences, shortening of linker, and splitting of second stem-loop in tracrRNA

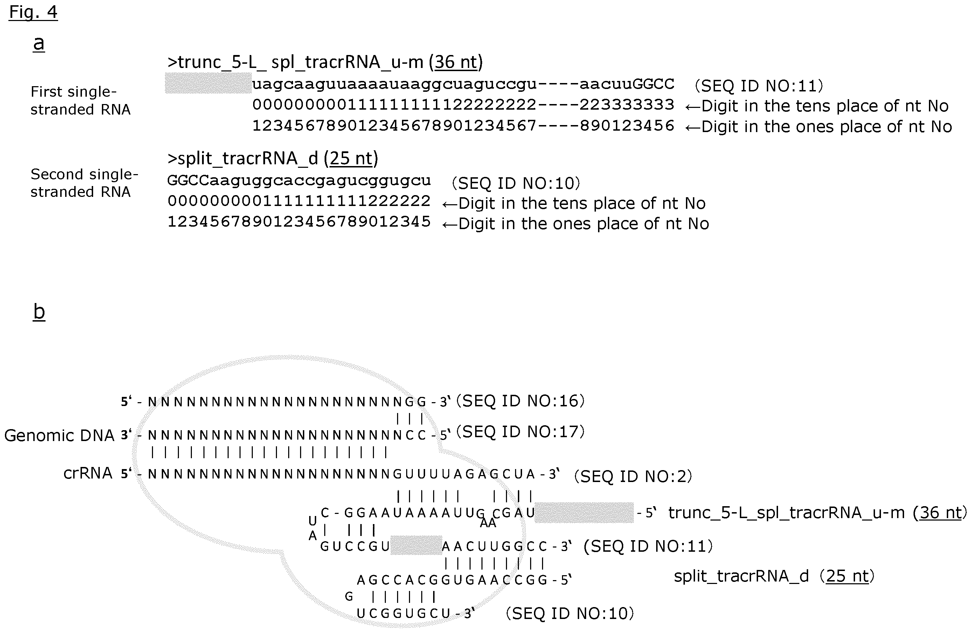

In the normal type of tracrRNA described in the above section (ii), nt Nos. 1 to 8 and nt Nos. 36 to 39 were deleted, split into nt Nos. 9 to 35, 40 to 44, and nt Nos. 49 to 69, 5'-GGCC-3' was added to a 3' side of a first single-stranded RNA located on the 5' side, and 5'-GGCC-3' was added to a 5' side of a second single-stranded RNA located on the 3' side. A sequence of a unit is shown in Fig. 4a, and the predicted structure of the unit is shown in Fig. 4b. The nucleotide sequence of the first single-stranded RNA is 5'-UAGCAAGUUAAAAUAAGGCUAGUCCGUAACUUGGCC-3' (SEQ ID NO: 11), and the nucleotide sequence of the second single-stranded RNA is 5'-GGCCAAGUGGCACCGAGUCGGUGCU-3' (SEQ ID NO: 10). By Watson-Crick base pairing, it is expected that nt Nos. 29, 30, 31, 32, 33, 34, 35, 36 of the first single-stranded RNA represented by SEQ ID NO: 11 in Fig. 4a will interact with nt Nos. 8, 7, 6, 5, 4, 3, 2, 1 of the second single-stranded RNA represented by SEQ ID NO: 10 in Fig. 4a, and by mismatch base pairing, it is expected that nt No. 28 of the first single-stranded RNA represented by SEQ ID NO: 11 in Fig. 4a will interact with nt No. 9 of the second single-stranded RNA represented by SEQ ID NO: 10 in Fig. 4a. The first "u" in SEQ ID NO: 11 corresponds to the 9th "u" in SEQ ID NO: 1 shown in Fig. 2, and the fifth "a" in SEQ ID NO: 10 corresponds to the 49th "a" in SEQ ID NO: 1 shown in Fig. 2. Synthesis of the RNA was performed by FASMAC.

### (viii) Splitting of first stem-loop and second stem-loop in tracrRNA

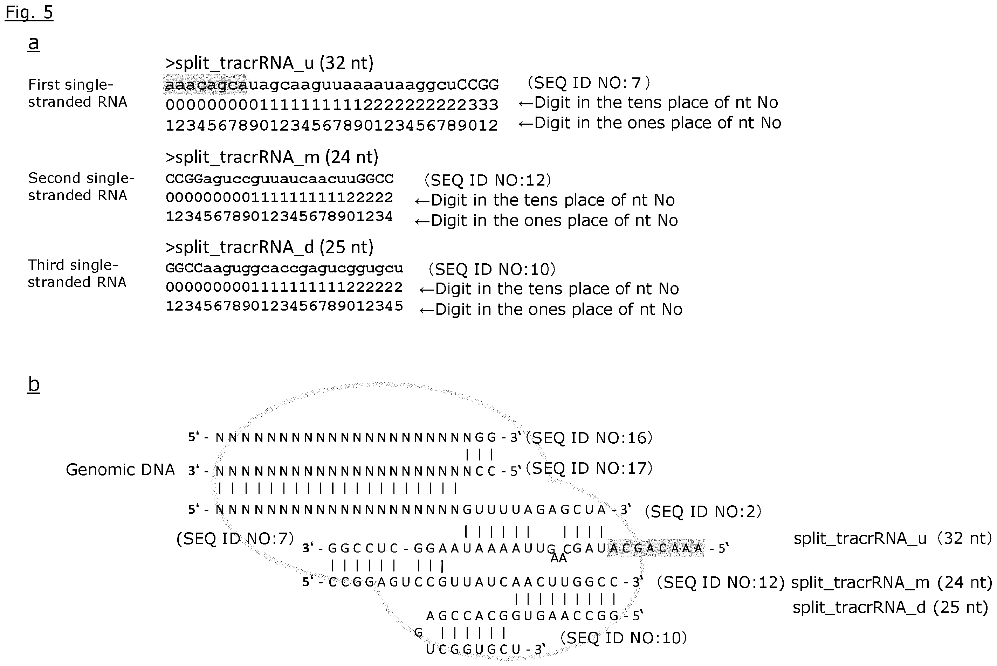

The normal type of tracrRNA described in the above section (ii) was split into nt Nos. 1 to 28, nt Nos. 29 to 44, and nt Nos. 49 to 69, 5'-CCGG-3' was added to the 3' side of the first single-stranded RNA located at the most 5' side, 5'-CCGG-3' was added to the 5' side of the second single-stranded RNA located at the middle, 5'-GGCC-3' was added to the 3' side of the second single-stranded RNA located at the middle, and 5'-GGCC-3' was added to the 5' side of the third single-stranded RNA located at the most 3' side. A sequence of a unit is shown in Fig. 5a, and the predicted structure of the unit is shown in Fig. 5b. The nucleotide sequence of the first single-stranded RNA is 5'-AAACAGCAUAGCAAGUUAAAAUAAGGCUCCGG-3' (SEQ ID NO: 7), the nucleotide sequence of the second single-stranded RNA is 5'-CCGGAGUCCGUUAUCAACUUGGCC-3' (SEQ ID NO: 12), and the nucleotide sequence of the third single-stranded RNA is 5'-GGCCAAGUGGCACCGAGUCGGUGCU-3' (SEQ ID NO: 10). By Watson-Crick base pairing, it is expected that nt Nos. 25, 26, 27, 28, 29, 30, 31, 32 of the first single-stranded RNA represented by SEQ ID NO: 7 in Fig. 5a will interact with nt Nos. 9, 8, 6, 5, 4, 3, 2, 1 of the second single-stranded RNA represented by SEQ ID NO: 12 in Fig. 5a, and nt Nos. 17, 18, 19, 20, 21, 22, 23, 24 of the second single-stranded RNA represented by SEQ ID NO: 12 in Fig. 5a will interact with nt Nos. 8, 7, 6, 5, 4, 3, 2, 1 of the third single-stranded RNA represented by SEQ ID NO: 10 in Fig. 5a. By mismatch base pairing, it is expected that nt No. 24 of the first single-stranded RNA represented by SEQ ID NO: 7 in Fig. 5a will interact with nt No. 10 of the second single-stranded RNA represented by SEQ ID NO: 12 in Fig. 5a, and nt No. 16 of the second single-stranded RNA represented by SEQ ID NO: 12 in Fig. 5a will interact with nt No. 9 of the third single-stranded RNA represented by SEQ ID NO: 10 in Fig. 5a. The first "a" in SEQ ID NO: 7 corresponds to the first "a" in SEQ ID NO: 1 shown in Fig. 2, the fifth "a" in SEQ ID NO: 12 corresponds to the 29th "a" in SEQ ID NO: 1 shown in Fig. 2, and the fifth "a" in SEQ ID NO: 10 corresponds to the 49th "a" in SEQ ID NO: 1 shown in Fig. 2. Synthesis of the RNA was performed by FASMAC.

### (ix) Shortening of 5' side sequences, splitting of first stem-loop, and splitting of second stem-loop in tracrRNA

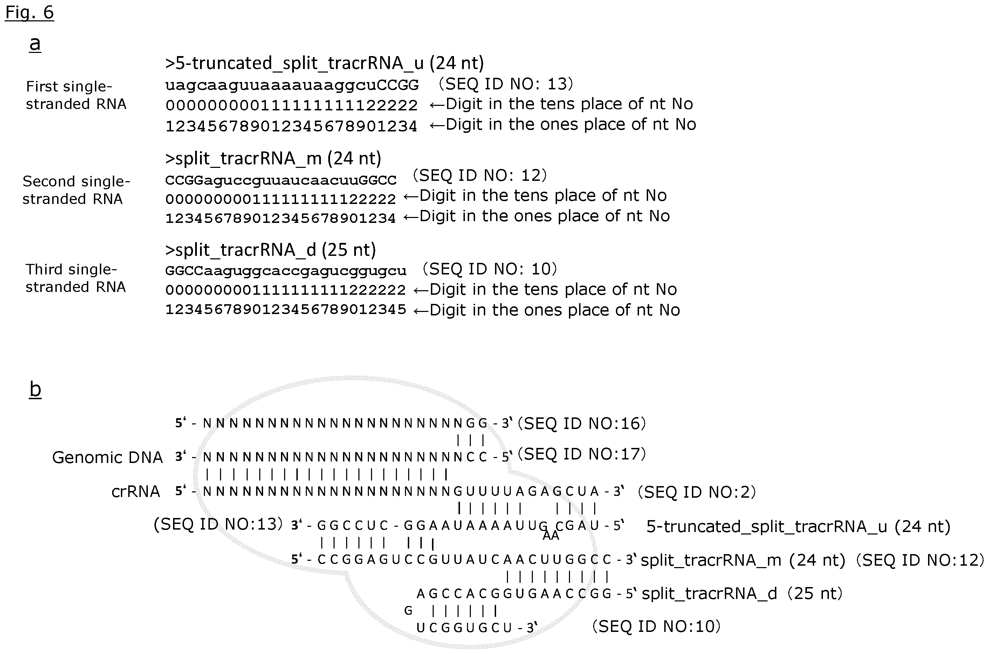

In the normal type of tracrRNA described in the above section (ii), nt Nos. 1 to 8 were deleted, split into nt Nos. 9 to 28, 29 to 44, and nt Nos. 49 to 69, 5'-CCGG-3' was added to the 3' side of the first single-stranded RNA located at the most 5' side, 5'-CCGG-3' was added to the 5' side of the second single-stranded RNA located at the middle, 5'-GGCC-3' was added to the 3' side of the second single-stranded RNA located at the middle, and 5'-GGCC-3' was added to the 5' side of the third single-stranded RNA located at the most 3' side. A sequence of a unit is shown in Fig. 6a, and the predicted structure of the unit is shown in Fig. 6b. The nucleotide sequence of the first single-stranded RNA is 5'-UAGCAAGUUAAAAUAAGGCUCCGG-3' (SEQ ID NO: 13), the nucleotide sequence of the second single-stranded RNA is 5'-CCGGAGUCCGUUAUCAACUUGGCC-3' (SEQ ID NO: 12), and the nucleotide sequence of the third single-stranded RNA is 5'-GGCCAAGUGGCACCGAGUCGGUGCU-3' (SEQ ID NO: 10). By Watson-Crick base pairing, it is expected that nt Nos. 17, 18, 19, 20, 21, 22, 23, 24 of the first single-stranded RNA represented by SEQ ID NO: 13 in Fig. 6a will interact with nt Nos. 9, 8, 6, 5, 4, 3, 2, 1 of the second single-stranded RNA represented by SEQ ID NO: 12 in Fig. 6a, and nt Nos. 17, 18, 19, 20, 21, 22, 23, 24 of the second single-stranded RNA represented by SEQ ID NO: 12 in Fig. 6a will interact with nt Nos. 8, 7, 6, 5, 4, 3, 2, 1 of the third single-stranded RNA represented by SEQ ID NO: 10 in Fig. 6a. By mismatch base pairing, it is expected that nt No. 16 of the second single-stranded RNA represented by SEQ ID NO: 13 in Fig. 6a will interact with nt No. 10 of the second single-stranded RNA represented by SEQ ID NO: 12 in Fig. 6a, and nt No. 16 of the second single-stranded RNA represented by SEQ ID NO: 12 in Fig. 6a will interact with nt No. 9 of the third single-stranded RNA represented by SEQ ID NO: 10 in Fig. 6a. The first "u" in SEQ ID NO: 13 corresponds to the 9th "u" in SEQ ID NO: 1 shown in Fig. 2, the fifth "a" in SEQ ID NO: 12 corresponds to the 29th "a" in SEQ ID NO: 1 shown in Fig. 2, and the fifth "a" in SEQ ID NO: 10 corresponds to the 49th "a" in SEQ ID NO: 1 shown in Fig. 2. Synthesis of the RNA was performed by FASMAC.

### II. In vitro cleavage assay

### (i) Preparation of substrate DNA

A region around the target sequence at slc45a2 locus was amplified by PCR from medaka genomic DNA using KOD FX (TOYOBO Co., Ltd.) and a forward primer 5'-GTCACTCTGTATTGGGGCGCCTCCT-3' (SEQ ID NO: 14) and a reverse primer 5'-CTTGGCCTGATCTCAGGGCTGGATG-3' (SEQ ID NO: 15). Synthesis of the primers was performed by Eurofins Genomics. An amplicon was purified using NucleoSpin Gel and PCR Clean-up (Takara Bio Inc.) and a DNA concentration was determined using NanoDrop (Thermo Fisher Scientific K.K.). A loading buffer was added to a solution containing 100 ng of the DNA, electrophoresis was performed on an agarose gel supplemented with Midori Green (Nippon Genetics Co., Ltd.) and bands were confirmed. Designed nucleotide length is 253 base pairs.

### (ii) Annealing of crRNA and tracrRNA unit

40 to 200 ng/µL (final concentration) of the crRNA targeting the medaka slc45a2 gene described in the above section I (i), and the first single-stranded RNA and second single-stranded RNA; or the first single-stranded RNA, second single-stranded RNA, and third single-stranded RNA described in the above sections I (ii) to (ix) were mixed in equimolar amounts. Number of moles of each single-stranded RNA constructing the tracrRNA was calculated on the basis of nucleotide length as follows.
(crRNA concentration ng/µL) x (nucleotide length of each single-stranded RNA) / 42

The mixture was heated at 95°C for 5 minutes using a thermal cycler, then lowered to 25°C at 0.1°C per second and stored at 4°C.

### (iii) In vitro cleavage assay

To the RNA solution annealed in the above section (ii), 0.5 to 2 µg/µL (final concentration) of Cas9 protein (FASMAC), 20 ng/µL (final concentration) of the substrate DNA prepared in the above section (i), and H buffer (Takara Bio Inc.) were added. The mixture was subjected to a reaction at 37°C for 60 minutes using the thermal cycler, followed by a heat treatment at 80°C for 5 minutes and stored at 4°C.

### (iv) Agarose gel electrophoresis

The cleavage product obtained in the above section (iii) was added with 1 µg/µL (final concentration) of RNaseA (NIPPON GENE CO., LTD.), reacted at 37°C for 30 minutes using the thermal cycler, subjected to a heat treatment at 80°C for 5 minutes to prepare samples for electrophoresis, and stored at 4°C. The samples were then added with the loading buffer and subjected to electrophoresis on a 3% agarose gel supplemented with Midori Green (Nippon Genetics Co., Ltd.), and bands were confirmed by LED irradiation. The substrate DNA that is not cleaved is expected to be 253 base pairs, and cleaved substrate DNA is expected to be 144 and 109 base pairs.

### (v) Results

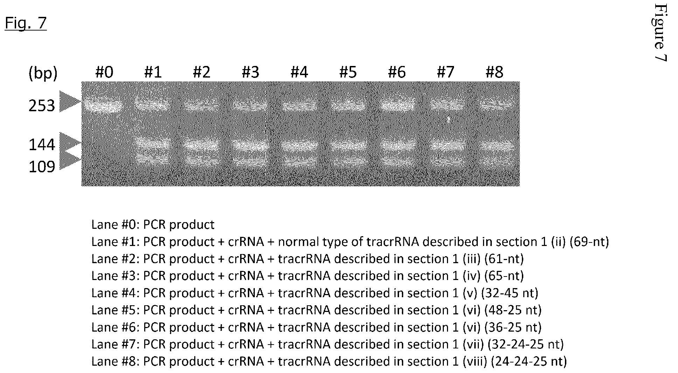

Fig. 7 shows results of the in vitro cleavage assay using the Cas9 protein from Streptococcus pyogenes as well as the crRNA described in the above section I (i) and the tracrRNA consisting of the first single-stranded RNA and second single-stranded RNA; or the first single-stranded RNA, second single-stranded RNA and third single-stranded RNA described in the above sections I (ii) to (ix) wherein the crRNA, and the tracrRNA was annealed. It was shown that each design produced cleavage products which appeared to be 114 and 109 base pairs, indicating having DNA cleavage activity.

### III. Introduction of mutation in fertilized egg of medaka

### (i) Obtaining fertilized egg of medaka

Sexually mature male and female medaka were allowed to coexist in an aquarium, and fertilized eggs were collected immediately after spawning.

### (ii) Microinjection for injecting CRISPR solution into cell

The fertilized eggs of medaka were obtained by the method described in the above section (i). By the method described in the above section II (ii), the crRNA targeting the slc45a2 gene locus of medaka, and the tracrRNA consisting of the first single-stranded RNA and second single-stranded RNA; or the first single-stranded RNA, second single-stranded RNA, and third single-stranded RNA were annealed, added with 0.5 to 2 µg/µL (final concentration) of the Cas9 protein (FASMAC), 50 ng/µL (final concentration) of GFP mRNA, 40 to 160 ng/µL (final concentration) of the crRNA, and 60 to 240 ng/µL (final concentration) of the tracrRNA, and the mixture was introduced into each fertilized egg at 1 to 5 nL per fertilized egg by microinjection. Then, the fertilized eggs were transferred to rearing water added with a small amount of methylene blue and maintained at 26°C. Next day, embryos with GFP fluorescent signals were selected as eggs correctly injected with CRISPR solution in the cells.

### (iii) Phenotypic analysis by genome editing

Four days after the microinjection, eyes of the medaka embryos were observed using a stereomicroscope (Olympus Corporation). If a mutation is introduced in the slc45a2 gene, melanophores do not construct, thus it is expected that part or all of the eye will be deficient in black pigment. Embryos that had been observed were maintained until the analysis described in the next section.

### (iv) Preparation of solution of fractured medaka

The fertilized eggs obtained in the above section (iii) were transferred to rearing water containing 1 × medaka ECM (0.1% NaCl, 0.003% KCl, 0.004% CaCl₂, 0.016% MgSO₄) with a small amount of methylene blue and maintained in an incubator at 26°C. After 5 to 10 days, the fertilized eggs were placed in 25 µL of alkaline buffer (25 mM NaOH, 0.2 mM EDTA), egg membranes thereof were broken with a 200 µL tip, and subjected to heat treatment at 95°C for 5 minutes using a thermal cycler. They were stored at 4°C as genomic DNA samples after neutralization by adding an equal volume (25 µL) of 40 mM Tris-HCl (pH 8.0).

### (v) Mutation analysis by heteroduplex mobility assay (HMA)

Five to ten days after the microinjection was conducted, the medaka embryos were fractured by the method described in the above section (iv). The regions described in the above section II (iii) were amplified by PCR and subjected to electrophoresis using MultiNA (SHIMADZU CORPORATION). If a mutation is introduced in the target region, it is expected that heteroduplexes will occur during the annealing process in the PCR, resulting in band shifts due to differences in the degree of mobility caused by physical characteristics. If the mutation is introduced more strongly, it is expected that band shifts will be detected more strongly due to the appearance of a large number of diverse mutations.

### (vi) Mutation analysis by Sanger sequencing method

Waveforms of nucleotide sequences were obtained by a Sanger sequencing method using the amplicons yielded by the PCR as described in the above section (iii). The Sanger sequencing method was performed by Eurofins Genomics. TIDE (Netherlands Cancer Institute) was used to estimate sequences and frequencies of mutant alleles.

### (vii) Results

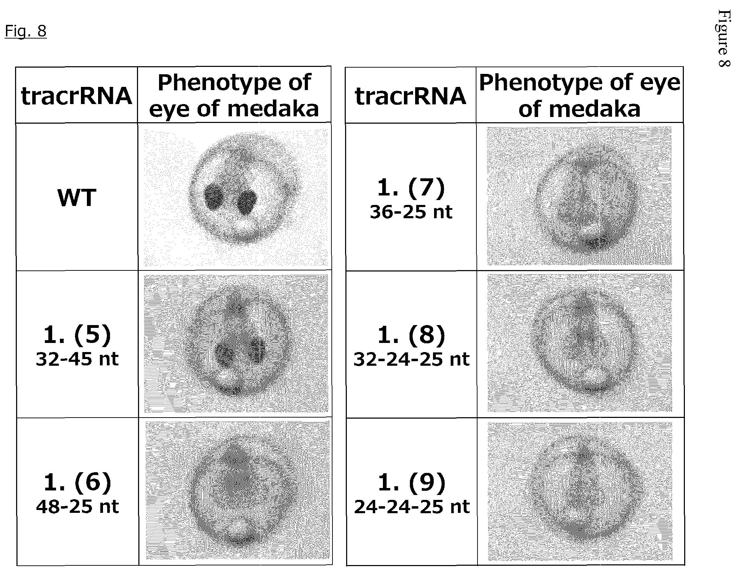

Fig. 8 shows observation results of the eyes of the medaka embryos injected with the Cas9 protein as well as the crRNA described in the above section I (i) and the tracrRNA consisting of the first single-stranded RNA and second single-stranded RNA; or the first single-stranded RNA, second single-stranded RNA, and third single-stranded RNA described in the above sections I (v) to (ix) wherein the crRNA and the tracrRNA was annealed. In all cases, a loss of black pigment in the eye in whole or in part was observed in several embryos.

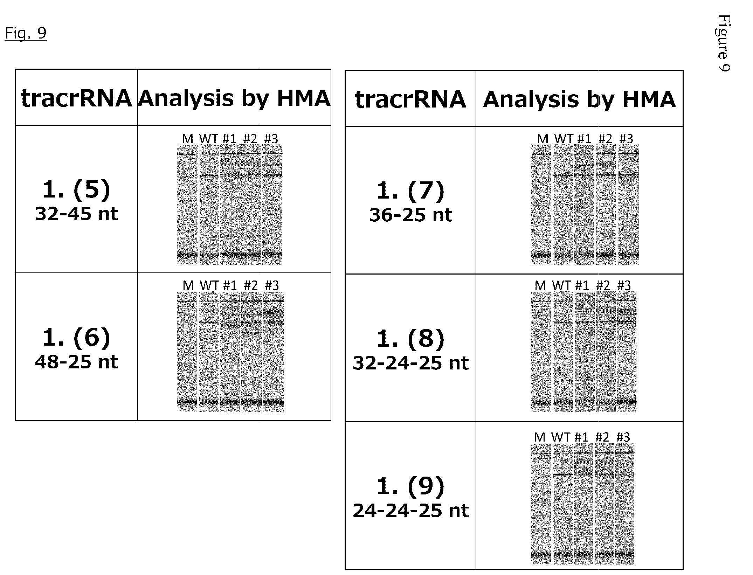

Fig. 9 shows results of HMA using lysates of the medaka embryos injected with the Cas9 protein as well as the crRNA described in the above section I (i) and the tracrRNA consisting of the first single-stranded RNA and second single-stranded RNA; or the first single-stranded RNA, second single-stranded RNA, and third single-stranded RNA described in the above sections I (v) to (ix) wherein the crRNA and the tracrRNA was annealed. In all cases, band shifts due to the introduction of the mutation were observed in several embryos.

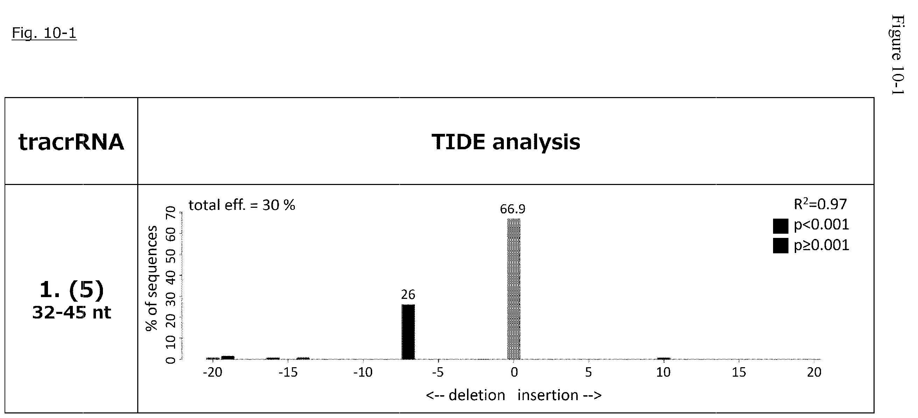

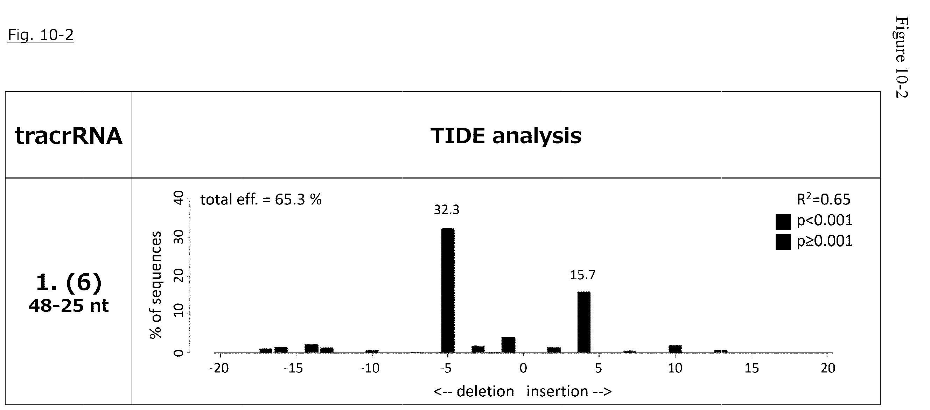

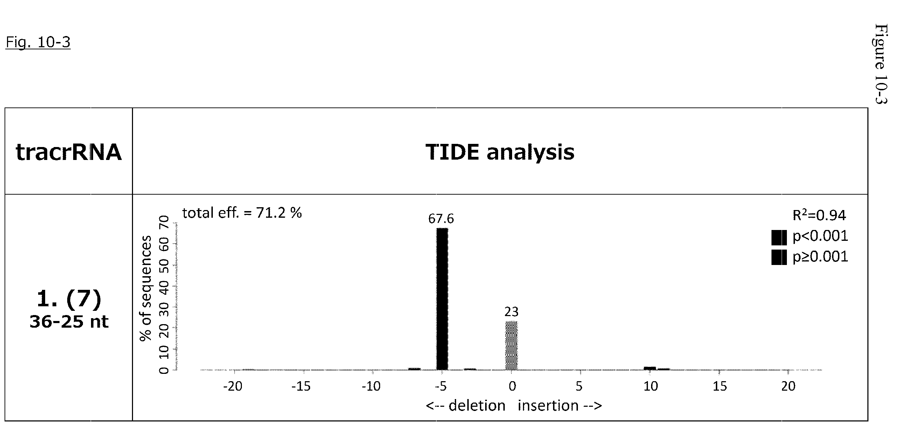

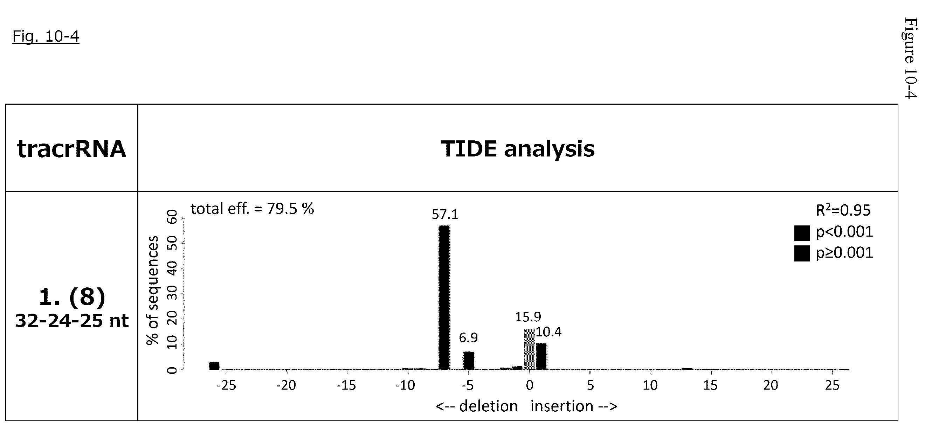

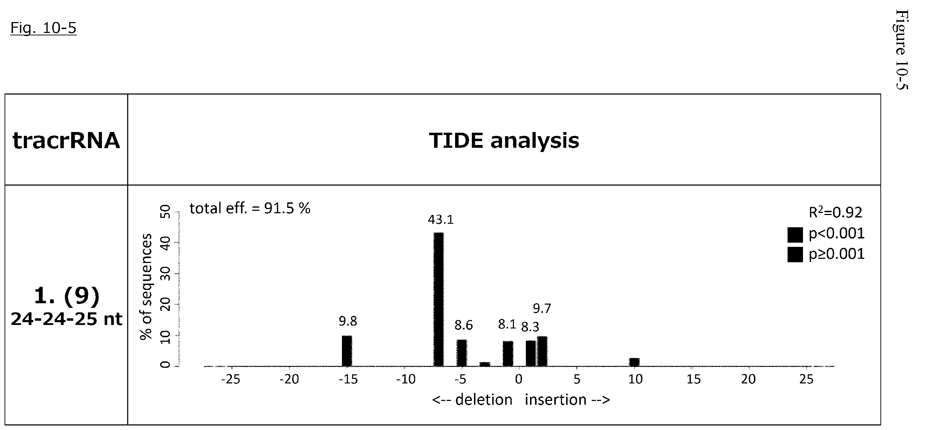

Figs. 10-1 to 10-5 show results of analysis by the mutation analysis by the Sanger sequencing method and TIDE using the lysates of the medaka embryos injected with the Cas9 protein as well as the crRNA described in the above section I (i) and the tracrRNA consisting of the first single-stranded RNA and second single-stranded RNA; or the first single-stranded RNA, second single-stranded RNA, and third single-stranded RNA described in the above sections I (v) to (ix) wherein the crRNA and the tracrRNA was annealed. In all cases, it was suggested that mutant alleles with deletion or insertion of bases were present in several embryos.

These results indicate that the tracrRNA functions as the tracrRNA are maintained even when the tracrRNA is split and, furthermore, when the nucleotide sequence is partially deleted from the tracrRNA.

Sequence Listing

## Claims

1. A tracrRNA unit comprising a first single-stranded RNA and a second single-stranded RNA for use in a CRISPR-Cas9 genome editing system wherein
the first single-stranded RNA and the second single-stranded RNA are not continuous,
the first single-stranded RNA has at least a first segment and a second segment, and
the first and second segments do not overlap, the first segment has a sequence complementary to a part of a crRNA, and the second segment has a sequence complementary to the second single-stranded RNA.

2. A tracrRNA unit comprising a first single-stranded RNA, a second single-stranded RNA, and a third single-stranded RNA for use in a CRISPR-Cas9 genome editing system wherein
the first single-stranded RNA, the second single-stranded RNA, and the third single-stranded RNA are not continuous,
the first single-stranded RNA has at least a first segment and a second segment,
the first and second segments do not overlap, the first segment has a sequence complementary to a part of a crRNA, and the second segment has a sequence complementary to the second single-stranded RNA,
the second single-stranded RNA has at least a first segment, and
the first segment of the second single-stranded RNA has a sequence complementary to the third single-stranded RNA.

3. The tracrRNA unit according to claim 1 or 2, wherein the crRNA and the first single-stranded RNA are linked in order.

4. The tracrRNA unit according to claim 2, wherein the crRNA, the first single-stranded RNA and the second single-stranded RNA are linked in order.

5. The tracrRNA unit according to claim 1, wherein at least one of the first single-stranded RNA and the second single-stranded RNA has a length of 39 nucleotides or less.

6. The tracrRNA unit according to claim 2, wherein at least one of the first single-stranded RNA, the second single-stranded RNA, and the third single-stranded RNA has a length of 39 nucleotides or less.

7. A genome editing method using
the tracrRNA unit according to any one of claims 1 to 6,
a crRNA and
a Cas9.
